# EUROPÄISCHE PATENTSCHRIFT

(11) **EP 0 316 893 B1**
(45) Veröffentlichungstag und Bekanntmachung des Hinweises auf die Patenterteilung: **19.01.1994**
(21) Anmeldenummer: 88119086.2
(22) Anmeldetag: 17.11.1988
(51) Int. Cl.: C07C 59/195, C07F 7/00, C07K 5/00

(54) **Kondensierte Acetessigsäureester-Titanchelate und Verfahren zu deren Herstellung**
Condensed acetoacetic ester-titanium chelates and process for their preparation
Chélates d'ester d'acide acéto-acétique-titane et leur procédé de préparation

(30) Priorität: 19.11.1987 DE 3739174
(43) Veröffentlichungstag der Anmeldung: 24.05.1989
(73) Patentinhaber: HÜLS AKTIENGESELLSCHAFT, 45764 Marl (DE)
(72) Erfinder: Barfurth, Dieter, D-5210 Troisdorf (DE); Nestler, Heinz, Dr., D-5210 Troisdorf (DE)

(56) Entgegenhaltungen:
- EP-A- 0 035 649
- EP-A- 0 140 102
- US-A- 4 551 544
- US-A- 4 643 913
- US-A- 4 743 474
- CHEMICAL ABSTRACTS, Band 105, 1986, Seite 125, Zusamenfassung Nr. 45159m, Columbus, Ohio, US; V.L. SIDOROV et al.: "Effect of organotitanium compounds on the waterproofing of chrome-tanned leathers by organosilicon products"

## Beschreibung

Titansäurealkylester sind seit langem bekannt als Vernetzer für hochmolekulare hydroxylgruppentragende Verbindungen, die so in Gele und plastische oder elastische Massen überführt werden können. Da die Niederalkylester der Titansäure im allgemeinen sehr rasch mit den genannten Hydroxylverbindungen reagieren, also beispielsweise sofort beim Vermischen der Komponenten oder bereits vor der Anwendung in den Vorratsbehältern, werden statt ihrer häufig Titanesterchelate eingesetzt, deren Reaktivität in gewünschter Weise vermindert ist. Bekannte Chelatbilder sind beispielsweise Acetessigsäureethylester, und ein gebräuchliches Titanesterchelat mit letzterer Verbindung ist Diisopropoxy-bis(ethylacetoacetato)-titan (I), hergestellt durch Reaktion von Tetraisopropyltitanat Ti(OC₃H₇)₄ mit 2 Äquivalenten Acetessigsäureethylester CH₃COCH₂COOC₂H₅. Diese Verbindung I fällt zunächst als Flüssigkeit an, häufig erstarrt sie aber, nicht nur beim Abkühlen, sondern auch bei Zimmertemperatur, und hat dann einen Schmelzpunkt von 25 bis 28° C.

Dieses Verhalten ist bei der Anwendung der Verbindung I häufig störend, erfordert sie doch in der Regel eine Vorrichtung zur Verflüssigung des Produktes vor einer Vermischung mit den anderen, normalerweise flüssigen Komponenten eines Reaktionsansatzes, und die dabei nötige Manipulation des Erwärmens und Homogenisierens.

Um diesem Nachteil abzuhelfen, ist in der US-PS 4,478,755 bereits vorgeschlagen worden, den bei der oben beschriebenen Reaktion zwischen dem Titanat und dem Acetessigester freigewordenen Isopropylalkohol vollständig oder teilweise abzudestillieren und die Fehlmenge durch andere Alkohole wie Methyl- oder Butylalkohol zu ersetzen, oder aber von einem Gemisch verschiedener Alkyltitanate auszugehen und auf diese Weise verschiedene Alkohole durch die Reaktion freizusetzen. Auf beide Arten soll es gelingen, kältefeste Lösungen des Titanchelats I in Alkoholen herzustellen.

EP-A-0 035 649 beschreibt kältestabile, katalytisch wirksame Lösung von Diisopropoxy-bis (2,4-pentan-dionato)-titan IV in Isopropanol.

Bei bestimmten Anwendungen solcher Titanchelate, z.B. als Kondensations- oder Härtungskatalysatoren in Silikonmassen, wie sie u.a. in der DE-PS 22 28 883 beschrieben sind, würde das Vorhandensein niederer Alkohole stören. Es bestand also die Aufgabe, die Verbindung I so abzuwandeln, daß ihre Kristallisierneigung reduziert, aber gleichzeitig ihre Reaktivität nicht merklich verändert wird.

Überraschenderweise zeigte sich, daß Kondensationsprodukte aus I diesen Anforderungen genügen. Solche Kondensationsprodukte bilden sich, indem man Diisopropoxy-bis(ethylacetoacetato)-titan (I) durch Zugabe von Wasser partiell hydrolysiert und den entstandenen Isopropylalkohol destillativ entfernt. Dabei sollte der Kondensationsgrad 2, wobei 2 Äquivalente I mit einem Äquivalent H₂O reagieren, nicht überschritten werden, da längerkettige Kondensate ihrerseits wieder zu Ausfällungen oder zum Festwerden bei Zimmertemperatur neigen. Versetzt man I mit weniger Wasser als dem Kondensationsgrad 2 entsprechend, so entsteht eine Mischung aus kondensiertem Produkt und unverändertem Ausgangschelat I, das sich bezüglich Kristallisationsneigung ebenfalls günstiger verhält als reines I. Bevorzugt ist daher die Umsetzung von 2 Mol eines Titan-Acetylacetat-Chelats mit 1 Mol Wasser, möglich ist auch die Umsetzung mit 0,5 bis 1,0 Mol Wasser.

Praktisch lassen sich die erfindungsgemäßen Titanesterchelate nach einem Eintopfverfahren herstellen, indem man Isopropyltitanat zunächst mit Acetessigsäureester und sofort anschließend mit Wasser reagieren läßt und schließlich den gebildeten Isopropylalkohol abdestilliert. Es ist auch möglich, die Reaktion mit Wasser zu einer späteren Zeit vorzunehmen.

Gegenstand der Erfindung sind daher die alkoholfreien Acetessigester-Chelate des Titans, deren herstellverfahren sowie deren Verwendung als Katalysatoren nach den Patentansprüchen.

Das durch Reaktion von 2 Mol I mit einem Mol Wasser hergestellte Dimere IA bleibt selbst bei -25° C über Wochen flüssig. Auch Animpfen mit I-Kristallen führt nicht zu spontanem Kristallisieren.

Ähnlich, wenn auch nicht so ausgeprägt, sind die Verhältnisse bei dem Reaktionsprodukt aus einem Mol Isopropyltitanat und einem Mol Acetessigsäureethylester, Tri-isopropoxy-ethylacetoacetato-titan (II). Aus diesem nach Abdestillieren des entstandenen Isopropanols zunächst flüssig anfallenden Produkt bilden sich bereits beim Stehen bei Raumtemperatur Kristalle, und beim Abkühlen auf +5° C kristallisiert das gesamte Produkt durch; es verflüssigt sich bei anschließendem Stehen bei Raumtemperatur auch nach 8 Std. in nur sehr geringem Ausmaß.

Versetzt man 2 Mol II mit einem Mol Wasser, was wegen der geringeren Hydrolysestabilität des Produktes nur in Verdünnung mit Isopropanol geschehen kann, fällt nach dem Abdestillieren des Isopropanols eine Flüssigkeit an, aus der auch nach längerem Stehen bei Raumtemperatur keine Kristalle ausfallen. Auch hier ist also das kondensierte Produkt (IIA) weniger kristallisierfreudig und damit besser handhabbar. Beim Abkühlen auf +5° C erstarrt die Flüssigkeit glasartig mit nur sehr wenigen kristallinen Bezirken und ist nach etwa einstündigem anschließenden Stehen bei Raumtemperatur wieder vollständig flüssig.

Allgemein können als Ausgangsstoffe Diisopropoxy-bis-(alkylaceto-acetato)-titan oder Triisopropoxy-alkylacetoacetato-titan verwendet werden, in denen die Alkylreste gleiche oder verschiedene Reste mit 2 bis 4 C-Atomen sind; bevorzugt ist der Ethylrest.

Bei der Kondensation wird je Titan eine Alkoxygruppe als Alkohol abgespalten, so daß eine Ti-O-Ti-Brücke entsteht und Produkte vom Kondensationsgrad 2 entstehen.

Vorteilhaft ist die Herstellung im Eintopfverfahren durch Umsetzung von Tetraalkyl-titanat mit 1 oder 2 Mol Acetessigester je Ti und anschließender Zugabe von Wasser und Abdestillation des freigesetzten und des ggf. zugesetzten Alkohols. Bei der Kondensation soll die Temperatur ca. 100° C nicht überschreiten.

Kondensierte Produkte der beschriebenen Art sind als chelatisierte Titanate dazu befähigt, Polymere mit entsprechenden funktionellen Gruppen zu vernetzen. Insbesondere lassen sich alkoholfreie Verbindungen wie IA in der Silikonchemie als Bestandteil von Härtungssystemen für Hydroxylendgruppen-tragende Silikone verwenden, die als Dichtmassen Bedeutung haben.

Die Beispiele dienen der näheren Erläuterung von Herstellung und Eigenschaften der erfindungsgemäßen Stoffe.

Der Kondensationsgrad von zwei bedeutet, daß zwei Ti-Atome durch eine Sauerstoffbrücke -0- verbunden sind. Die Formel der neuen Acetessigsäueeester-Chelate ist: 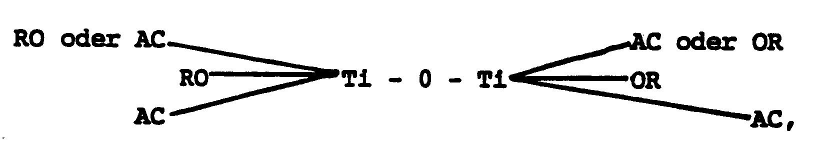
worin AC den Acetoacetato-Rest (Acetessigsäureester-Ligand) und OR Alkoxy mit R von 1 bis 4 C-Atomen bedeutet und die Summe von AC und OR je Ti-Atom drei ist, d.h. ein oder zwei Acetessigsäureester-Liganden und eine sich zur Zahl drei ergänzende Zahl von Alkoxygruppe je Ti-Atom enthalten sind.

### Beispiel 1

### Diisopropoxy-bis(ethylacetoacetato)-titan (I)

In einem 10-l-Kolben mit Rührer, Thermometer, Tropftrichter und Rückflußkühler werden 3.408 g (12 Mol) Tetraisopropyltitanat vorgelegt und unter Rühren innerhalb 30 min mit 3.120 g (24 Mol) Acetessigsäureethylester versetzt, wobei die Temperatur 75° C nicht überschreiten soll. Nach einstündigem Rühren bei ca. 70° C wird der Rückflußkühler gegen eine Destillationsbrücke ausgetauscht und Isopropanol abdestilliert (25 mm Hg/max. 90° C Sumpftemperatur); 1.392 g Isopropanol = 98,2 % d. Th.

Im Sumpf verbleiben 5.114 g Produkt I = 100,5 % d. Th.
- Aussehen:: orangerote Flüssigkeit, die nach Stunden oder Tagen kristallisiert.
- Schmelzpunkt:: ca. 28° C,
- Dichte (35° C):: ca. 1,09 g/ml,
- Viskositit (35° C):: ca. 20 mPa.s,
- Brechungszahl (35° C):: ca. 1.513.

### Beispiel 2

### Kondensationsprodukt IA

In einem 4-l-Kolben mit Rührer, Thermometer, Tropftrichter und Rückflußkühler werden 1.136 g (4 Mol) Tetraisopropyltitanat vorgelegt und unter Rühren innerhalb 10 min mit 1.040 g (8 Mol) Acetessigsäureethylester versetzt, wobei die Temperatur 75° C erreicht. Nach schneller Zugabe von 36 g (2 Mol) Wasser wird 1 Std Zum Rückfluß erhitzt (Sumpftemperatur 88° C), dann der Rückflußkühler gegen eine Destillationsbrücke ausgetauscht und der vorhandene Alkohol im Vakuum (gegen Ende 25 mm Hg/90° C Sumpftemperatur) abdestilliert; 659 g Alkohol = 92 % d. Th. Im Sumpf verbleiben 1.554 g Produkt IA = 104 % d. Th. (bedingt durch teilweise Umwandlung des Ethyl- in den Isopropylester).
Aussehen: rote Flüssigkeit,
Dichte (20° C): ca. 1,15 g/ml,
Viskosität (20° C): ca. 55 mPa.s,
Brechungszahl (20° C): ca. 1,526,

| | | | |
|---|---|---|---|
| Elementaranalyse: | C 48,2 %, | H 7,3 %, | Ti 12,3 %, |
| (Theorie gemittelte Werte: | C 49,6 %, | H 7,0 %, | Ti 12,4 %), |

Verhalten bei Abkühlung: bei tagelangem Stehen bei -25° C bleibt das Produkt flüssig, auch nach Animpfen mit I-Kristallen.

### Beispiel 3

### Triisopropoxy-ethylacetoacetato-titan (II)

In einem 1-l-Kolben mit Rührer, Thermometer, Tropftrichter und Rückflußkühler werden 426 g (1,5 Mol) Tetraisopropyltitanat vorgelegt und unter Rühren innerhalb von 20 min mit 195 g (1,5 Mol) Acetessigsäureethylester versetzt, wobei die Temperatur 57° C erreicht. Nach einstündigem Rühren bei 75 bis 80° C wird der Rückflußkühler gegen eine Destillationsbrücke ausgetauscht und im Vakuum (am Ende 12 mm Hg/72° C Sumpftemperatur) das Isopropanol abdestilliert (93 g = ca. 100 % d. Th.).

Im Sumpf verbleiben 525 g = 98,9 % d. Th. Produkt II in Form einer orangen Flüssigkeit, aus der sich nach einigen Stunden Stehen Kristalle absondern.
Dichte (20° C): ca. 1.059,
Viskosität (20° C): ca. 130 mPa.s,
Brechungszahl (20° C): ca. 1.510,
Verhalten bei Abkühlung: bei +5 °C kristallisiert die Flüssigkeit.

| Formel: | C₂₄ | H₄₆0₁₁ | Ti₂ |
|---|---|---|---|
| ber.: | C47.5 % | H 7.6 % | Ti 15.8 % |
| gefunden: | C47 % | H 7.8 % | Ti 15.6 % . |

### Beispiel 4

### Kondensationsprodukt IIA

In einem 1-l-Kolben mit Rührer, Thermometer, Tropftrichter und Rückflußkühler werden 284 g (1 Mol) Tetraisopropyltitanat vorgelegt und unter Rühren innerhalb 10 min mit 130 g (1 Mol) Acetessigsäureethylester versetzt, wobei die Temperatur 59° C erreicht. Nach kurzem Aufheizen auf 75° C wird auf 50° C abgekühlt und die Mischung aus 9 g (0,5 Mol) Wasser und 240 g Isopropanol innerhalb 30 min zugesetzt. Nach 1 Std Rühren bei 70° C wird der Alkohol im Vakuum abdestilliert (am Ende 15 mm Hg/57° C Sumpftemperatur), 361 g = 100 % d. Th.

Im Sumpf verbleiben 303 g Produkt IIA = 100 % d. Th. in form einer orangen Flüssigkeit.
- Dichte (20° C):: ca. 1.129,
- Viskosität (20° C):: ca. 220 mPa.s,
- Brechungszahl (20° C):: ca. 1.533,
- Verhalten bei Abkühlung:: nach mehrstündigem Stehen bei +5° C erstarrt die Flüssigkeit glasig unter Bildung vereinzelter kristallisierter Bezirke. Nach 40 min Stehen bei Raumtemperatur ist das Produkt wieder flüssig.

### Beispiel 5

### Vernetzung von Silikonpolymeren

Bei der Verwendung von Silikonpolymeren, die endständige Hydroxylgruppen tragen, in Dichtmassen müssen - unter vielen anderen - auch zwei Kriterien beachtet werden, die direkt mit der Vernetzung der Einzelmoleküle zu hochpolymeren Elastomeren zusammenhängen: die Stabilität und möglichst vollständige Unveränderlichkeit des Produktes im Vorratsgefäß unter Luftabschluß und die ausreichend schnelle Härtung bei Zutritt der Luftfeuchtigkeit.

Um zu prüfen, ob das erfindungsgemäße Produkt IA diesen Kriterien standhält, wurden folgende Testmethoden ausgewählt:
**Hautbildungstest:** Zeitdauer vom Ausbringen der Silikonmasse in dünner Schicht auf eine Unterlage bis zu Hautbildung (kein Kleben bei Berührung).
**Fließtest:** Pulverflaschen werden zu etwa 1/3 mit den Silikonmassen gefüllt, fest verschlossen und umgelegt. Gemessen wird die Zeitdauer, die vom Aufrichten der Flasche an vergeht, bis der Flascheninhalt in seine ursprüngliche Lage zurückgeflossen ist.
**Testmaterialien:** Handelsübliches Hydroxyendgruppen-tragendes Silikonpolymer (15.000 mPa.s);
Additiv 1: 20 gew.-%ige Lösung von Produkt IA in Toluol;
Additiv 2: 20 gew.-%ige Lösung einer 1 : 2-Mischung aus Methyl-tris-(ethylmethylketoximo)-silan und Produkt IA in Toluol;
Additiv 3: 50 gew.-%ige Lösung der vorstehenden Mischung in Toluol.
**Versuchsansätze:** Das Silikonpolymer wird mit der in nachstehender Tabelle aufgeführten Menge der Additive 1 bis 3 schnell verrührt und sofort in einem Vorratsgefäß luftdicht verschlossen.
Ergebnisse:

| Additiv: | Menge: Gew.-% | Hautbildungszeit: | Fließzeit nach | | | |
|---|---|---|---|---|---|---|
| | | | 1Std | 1Tag | 2Tagen | 3Wochen |
| 1 | 3 | 6 min | 7min | 8min | ./. | 7min |
| 2 | 3 | 1Std 10min | 3min | 4,5min | ./. | 8min |
| 3 | 2 | 14min | ./. | 20min | 60min | ./. |
| Vergleich Silikon ohne Additiv | | | 10 s | 15 s | ./. | 10 s |

## Patentansprüche

1. Alkoholfreie Acetessigsäureester-Chelate des Titan-IV mit verbessertem Kälteverhalten, dadurch gekennzeichnet, daß sie kondensierte Titanchelate mit dem Kondensationsgrad 2 der Formel, 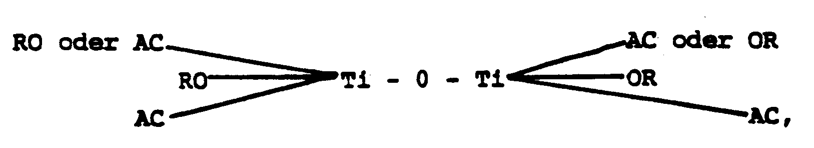 worin AC den Acetoacetato-Rest und OR Alkoxy mit R von 1 bis 4 C-Atomen bedeutet und die Summe von AC und OR je Ti-Atom drei ist, darstellen.

2. Titanchelate nach Anspruch 1, dadurch gekennzeichnet, daß sie ein der zwei Acetessigsäureester-Liganden und eine sich zu drei ergänzende Zahl von Alkoxygruppen pro Ti-Atom, vorzugsweise Isopropoxygruppen enthalten.

3. Verfahren zur Herstellung von Titanchelaten nach einem der Ansprüche 1 oder 2, dadurch gekennzeichnet, daß sie aus den monomeren Ausgangsstoffen Diisopropoxy-bis(ethylacetoacetato)-titan oder Triisopropoxy-ethylacetoacetato-titan durch Reaktion mit Wasser und Abdestillieren des freigesetzten und bei Herstellung aus Triisopropoxy-ethylacetatotitan zugesetzten Alkohols hergestellt werden.

4. Verfahren zur Herstellung von Titanchelaten nach einem der Ansprüche 1 oder 2, dadurch gekennzeichnet, daß sie in einem Eintopfverfahren, ausgehend von Tetraisopropyltitanat durch aufeinander folgende Umsetzungen mit Acetessigsäureethylester und mit Wasser und anschließendes Abdestillieren des freigesetzten und des ggf. zugesetzten Alkohols hergestellt werden.

5. Verfahren nach einem der Ansprüche 3 oder 4, dadurch gekennzeichnet, daß je 2 Mol Titanester-Einsatzmenge 0,5 bis 1 Mol Wasser, vorzugsweise 1 Mol Wasser zugesetzt wird.

6. Verwendung der Titanchelate nach einem der Ansprüche 1 bis 5 als Katalysatoren für die Vernetzung von Polymeren mit funktionellen Gruppen, insbesondere von Hydroxylendgruppen-tragenden Silikonen.

## Claims

1. Alcohol-free acetoacetic acid ester chelates of titanium-IV with improved cold behaviour, **characterised in that** they represent condensed titanium chelates with the degree of condensation 2 of the formula 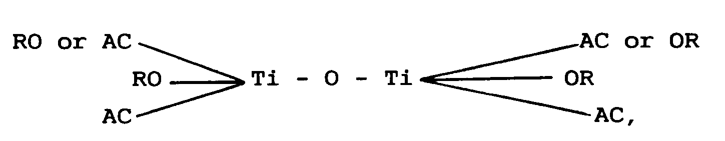 wherein AC signifies the acetoacetato residue and OR, alkoxy with R of 1 to 4 C atoms and the sum of AC and OR is 3 per Ti atom.

2. Titanium chelates according to claim 1, **characterised in that** they contain one or two acetoacetic acid ester ligands and a number of alkoxy groups, preferably isopropoxy groups, per Ti atom increasing to 3.

3. Process for the preparation of titanium chelates according to one of claims 1 or 2, **characterised in that** they are produced from the monomeric starting materials diisopropoxy-bis(ethylacetoacetato)-titanium or triisopropoxy-ethylacetoacetato-titanium by reaction with water and distillation off of the liberated alcohol and alcohol added in the production from triisopropoxy-ethylacetato-titanium.

4. Process for the production of titanium chelates according to one of claims 1 or 2, **characterised in that** they are produced in a one pot process starting from tetraisopropyltitanium by reactions following one another with acetoacetic acid ethyl ester and with water and subsequent distillation off of the alcohol liberated and the alcohol optionally added.

5. Process according to one of claims 3 or 4, **characterised in that** 0.5 to 1 mol of water, preferably 1 mol of water, is added per 2 mol of the amount of titanium ester employed.

6. Use of the titanium chelates according to one of claims 1 to 5 as catalysts for the cross linking of polymers with functional groups, in particular of hydroxyl end group-carrying silicones.

## Revendications

1. Chélates du titane-IV avec des esters de l'acide acétoacétique sans alcool, ces chélates ayant un comportement amélioré à froid, caractérisés en ce qu'ils représentent des chélates de titane condensés, ayant le degré de condensation 2 et en ce qu'ils répondent à la formule: 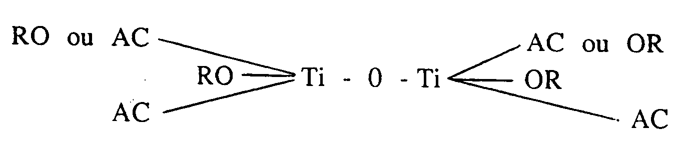 dans laquelle AC représente le reste acétoacétato et OR un reste alcoxy dans lequel R comporte 1 à 4 atomes de carbone, et la somme de AC et de OR vaut trois pour chaque atome de Ti.

2. Chélates de titane selon la revendication 1, caractérisés en ce qu'ils contiennent un ou deux ligands esters d'acide acétoacétique et un nombre, se complétant à trois, de groupes alcoxy par atome de Ti, avantageusement des groupes isopropoxy.

3. Procédé de préparation des chélates de titane selon l'une des revendications 1 ou 2, caractérisé en ce qu'on les prépare à partir des substances monomères de départ, le diisopropoxy-bis(éthylacétoacétato)-titane ou le triisopropoxy-éthylacétoacétato-titane, par réaction avec l'eau et départ par distillation de l'alcool libéré et de l'alcool ajouté lors de la préparation à partir du triisopropoxy-éthylacétato titane.

4. Procédé de préparation des chélates de titane selon l'une des revendications 1 ou 2, caractérisés en ce qu'on les prépare dans un procédé utilisant un seul réacteur, à partir de titanate de tétraisopropyle par des réactions successives avec l'acétoacétate d'éthyle et avec l'eau, puis départ par distillation de l'alcool libéré et de l'alcool éventuellement ajouté.

5. Procédé selon l'une des revendications 3 ou 4, caractérisé en ce que, pour chaque quantité introduite de 2 moles d'esters de titane, on ajoute 0,5 à 1 mole d'eau, avantageusement 1 mole d'eau.

6. Utilisation des chélates de titane selon l'une des revendications 1 à 5 comme catalyseurs pour la réticulation de polymères comportant des groupes fonctionnels, notamment des silicones comportant des groupes hydroxyles terminaux.
